# EUROPEAN PATENT APPLICATION

(11) **EP 4 653 547 A1**
(43) Date of publication of application: **26.11.2025**
(21) Application number: 24382538.7
(22) Date of filing: 20.05.2024
(51) Int. Cl.: C12Q 1/6883

(54) **IN VITRO METHODS FOR THE DIAGNOSIS OF MIGRAINE AND FOR THE DIFFERENTIAL DIAGNOSIS OF CHRONIC AND EPISODIC MIGRAINE**

(71) Applicant: Universidad Autónoma de Madrid, 28049 Madrid (ES); Fundación para la Investigación Biomédica del Hospital Universitario La Princesa, 28006 Madrid (ES); Universidad De Valladolid, 47002 Valladolid (ES); Gerencia Regional de Salud de Castilla y León, 47007 Valladolid (ES)
(72) Inventor: Sánchez Madrid, Francisco, 28049 Madrid (ES); Vivancos Mora, José, 28049 Madrid (ES); González Martínez, Alicia, 28006 Madrid (ES); De la Fuente, Hortensia, 28006 Madrid (ES); Gago-Veiga, Ana Beatriz, 28006 Madrid (ES); Planchuelo Gómez, Álvaro, 47002 Vallado (ES); Guerrero Peral, Ángel Luis, 47002 Va (ES)
(74) Representative: Hoffmann Eitle

(57) **Abstract**

The present invention refers to the medical field. Particularly, the present invention refers to an *in vitro* method for the diagnosis of migraine which comprises based on the assessment of the expression level of miR-375 in a biological sample obtained from a subject. It also refers to an *in vitro* method for the differential diagnosis of chronic and episodic migraine based on the assessment of the expression level of miR-375 in a biological sample obtained from a subject. It also refers to the *in vitro* use of the expression level of miR-375 for the diagnosis of migraine, or for the differential diagnosis of chronic and episodic migraine. In a preferred embodiment, the assessment of the expression level of miR-375 is combined with the assessment of other miRNAs or MRI parameters.

## Description

### FIELD OF THE INVENTION

The present invention refers to the medical field. Particularly, the present invention refers to an *in vitro* method for the diagnosis of migraine which comprises based on the assessment of the expression level of miR-375 in a biological sample obtained from a subject. It also refers to an *in vitro* method for the differential diagnosis of chronic and episodic migraine based on the assessment of the expression level of miR-375 in a biological sample obtained from a subject. It also refers to the *in vitro* use of the expression level of miR-375 for the diagnosis of migraine, or for the differential diagnosis of chronic and episodic migraine. In a preferred embodiment, the assessment of the expression level of miR-375 is combined with the assessment of other miRNAs or magnetic resonance imaging (MRI) parameters.

### STATE OF THE ART

Migraine is one of the most disabling medical conditions worldwide, diagnosed based on headache characteristics and associated symptoms. Moreover, it is a genetically driven chronic neurological disease among the most disabling neurological disorders of the Global Burden of Disease Study. Currently, the only criterion to distinguish between migraine types is the number of headache days per month, considering chronic migraine (CM) as 15 or more episodes per month, presenting at least eight of them migraine features, and episodic migraine (EM) otherwise, with no inclusion of biomarkers in the diagnosis to date.

On one hand, micro RNAs (miRNA) are small non-coding RNAs of approximately 20 nucleotides, highly stable, that regulate gene expression at the post-transcriptional level by inhibiting mRNA expression. They are quantifiable particles, making them potential measurement tools for different pathological processes that could facilitate disease monitoring, therapeutic decision-making, and personalized prognosis. In recent years, the potential role of miRNAs as biomarkers for migraine has been suggested. Differentially expressed miRNAs (DE miRNA) have been detected to date in migraine patients and controls in plasma and Peripheral blood mononuclear cells (PBMC). Several pilot studies have described miRNAs as potential peripheral biomarkers of migraine in plasma and PBMC. Moreover, serum extracellular vesicles miRNA profiles have been employed to assess acupuncture effects on migraine patients.

On the other hand, processing magnetic resonance imaging (MRI) data, differences between EM and CM have been found in numerous white matter tracts. In multiple studies, also using diffusion-weighted MRI data, multiple differences have been identified in white matter between patients with migraine and healthy subjects. Regarding gray matter, lower gray matter volume, surface area and cortical thickness, together with higher cortical curvature, have been identified in patients with migraine compared to healthy controls (HC), considering the area a potential biomarker between CM and EM. Changes in surface area in adults have been linked to genetic variations occurring during fetal development. However, cortical thickness changes mostly occur after birth. Additionally, relationships between cortical thickness and loci near genes involved in differentiation, migration, cellular adhesion, and myelination have also been found.

To date, no comprehensive study has been conducted simultaneously differentiating patterns with various MRI modalities in relation to epigenetic biomarkers such as serum extracellular vesicles miRNA profiles of migraineurs.

There is an unmet medical need for the development of improved methods for the diagnosis of migraine, as well as for the differential diagnosis of CM and EM. The present invention is focused on solving this problem, and *in vitro* methods for the diagnosis of migraine and for the differential diagnosis of CM and EM are herein provided.

### DESCRIPTION OF THE INVENTION

### Brief description of the invention

As explained above, the present invention refers to the medical field. Particularly, the present invention refers to an *in vitro* method for the diagnosis of migraine which comprises based on the assessment of the expression level of miR-375 in a biological sample obtained from a subject. It also refers to an *in vitro* method for the differential diagnosis of chronic and episodic migraine based on the assessment of the expression level of miR-375 in a biological sample obtained from a subject. It also refers to the *in vitro* use of the expression level of miR-375 for the diagnosis of migraine, or for the differential diagnosis of chronic and episodic migraine. In a preferred embodiment, the assessment of the expression level of miR-375 is combined with the assessment of other miRNAs or MRI parameters.

The inventors of the present invention performed a pilot study to identify differentially expressed miRNAs in peripheral blood in relation to changes in brain structure in patients with migraine. Through the analysis of the miRNA profiles in serum extracellular vesicles from healthy controls, and subjects with chronic and episodic migraine, the inventors of the present invention identified five miRNAs that could be potentially used as biomarkers for the diagnosis of migraine, CM, EM and/or for the differential diagnosis of CM and EM **(Table 17**). These miRNAs are: miR-375, miR-200a-3p, miR-141-3p, miR-454-3p and miR-16-2-3p.

Among them, miR-375 provided an AUC = 0.785 when used to diagnose migraine and an AUC = 0.61 when used for the differential diagnosis of CM and EM.

The combination of miR-375 with an additional miRNA selected from miR-200a-3p, miR-141-3p, miR-454-3p and/or miR-16-2-3p provided:
- an improved diagnostic capacity to diagnose migraine than the use of mi-375 alone, which the exception of the combination of miR-375 and miR-141-3p, which provided the same capacity to diagnose migraine than miR-375 alone **(Table 18**).
- an improved diagnostic capacity to differentiate CM and EM than the use of miR-375 alone, which the exception of the combination of miR-375 and miR-141-3p, which provided a lower AUC than that provided by miR-375 alone **(Table 18).**

The inventors also investigated the diagnostic capacity of the above-mentioned miRNAs in combination with MRI measurements. The inventors found that that the use of a miRNA selected from miR-375, miR-200a-3p, miR-141-3p, miR-454-3p and/or miR-16-2-3p in combination with the surface area of the right superior frontal gyrus measured by MRI provided:
- an improved diagnostic capacity to diagnose migraine than the use of each of the above-mentioned miRNAs alone, with the exception of miR-16-2-3p **(Table 19).**
- an improved diagnostic capacity to differentiate CM and EM than the use of each of the above-mentioned miRNAs alone, with the exception of miR-16-2-3p **(Table 19).**

In particular, the combination of miR-375 with the surface area of the right superior frontal gyrus measured by MRI provided:
- an improved diagnostic capacity to diagnose migraine (AUC = 0.825) than the use of miR-375 alone (AUC = 0.785, **Table 19).**
- an improved diagnostic capacity to differentiate CM and EM (AUC = 0.87) than the use of each of miR-375 alone (AUC = 0.61, **Table 19).**

In addition, the combination of miR-375, with an additional miRNA selected from selected from miR-200a-3p, miR-141-3p, miR-454-3p and/or miR-16-2-3p, with the surface area of the right superior frontal gyrus measured by MRI further improved the diagnostic capacity to diagnose migraine and the diagnostic capacity to differentiate CM and EM **(Table 20).**

The first aspect of the invention refers to an *in vitro* method for the diagnosis of migraine which comprises: (a) assessing the expression level of miR-375 in a biological sample obtained from a subject, b) wherein the identification of a lower expression level, as compared with a pre-established threshold value determined in subjects that do not suffer from migraine or by comparing subjects that suffer from migraine with subjects that do not suffer from migraine, is an indication that the subject suffers from migraine.

In a preferred embodiment, the *in vitro* method comprises: (a) assessing the expression level of miR-375 in combination with the expression level of a miRNA selected from: miR-200a-3p, miR-141-3p, miR-454-3p and/or miR-16-2-3p in a biological sample obtained from a subject, b) wherein the identification of a lower expression level, as compared with a pre-established threshold value determined in subjects that do not suffer from migraine or by comparing subjects that suffer from migraine with subjects that do not suffer from migraine, is an indication that the subject suffers from migraine.

In a preferred embodiment, the *in vitro* method comprises: (a) assessing the expression level of the following combinations of miRNAs: miR-375 and miR-200a-3p, miR-375 and miR-454-3p; and/or miR-375 and miR-16-2-3p in a biological sample obtained from a subject, b) wherein the identification of a lower expression level of the combination of miR-375 and miR-200a-3p, miR-375 and miR-454-3p; and/or miR-375 and miR-16-2-3p, as compared with a pre-established threshold value determined in subjects that do not suffer from migraine or by comparing subjects that suffer from migraine with subjects that do not suffer from migraine, is an indication that the subject suffers from migraine.

In a preferred embodiment, the *in vitro* method further comprises assessing the area of the right superior frontal gyrus, preferably by MRI, wherein a lower surface area of the right superior frontal gyrus, as compared with a pre-established threshold value determined in subjects that do not suffer from migraine or by comparing subjects that suffer from migraine with subjects that do not suffer from migraine, is an indication that the subject suffers from migraine.

In a preferred embodiment, the migraine is chronic migraine or episodic migraine.

The second aspect of the invention refers to an *in vitro* method for the differential diagnosis of chronic and episodic migraine which comprises: (a) assessing the expression level of miR-375 in a biological sample obtained from a subject, b) wherein the identification of a lower expression level, as compared with a pre-established threshold value determined in subjects that suffer from episodic migraine or by comparing subjects that suffer from episodic and chronic migraine, is an indication that the subject suffers from chronic migraine, and c) wherein the identification of a higher expression level, as compared with a pre-established threshold value determined in subjects that suffer from chronic migraine or by comparing subjects that suffer from episodic and chronic migraine, is an indication that the subject suffers from episodic migraine.

In a preferred embodiment, the *in vitro* method comprises: (a) assessing the expression level of miR-375 in combination with the expression level of a miRNA selected from: miR-200a-3p, miR-141-3p, miR-454-3p and/or miR-16-2-3p in a biological sample obtained from a subject, b) wherein the identification of a lower expression level, as compared with a pre-established threshold value determined in subjects that suffer from episodic migraine or by comparing subjects that suffer from episodic and chronic migraine, is an indication that the subject suffers from chronic migraine, and c) wherein the identification of a higher expression level, as compared with a pre-established threshold value determined in subjects that suffer from chronic migraine or by comparing subjects that suffer from episodic and chronic migraine, is an indication that the subject suffers from episodic migraine.

In a preferred embodiment, the *in vitro* method comprises: a) assessing the expression level of the following combinations of miRNAs: miR-375 and miR-200a-3p, miR-375 and miR-454-3p; and/or miR-375 and miR-16-2-3p in a biological sample obtained from a subject, b) wherein the identification of a lower expression level, as compared with a pre-established threshold value determined in subjects that suffer from episodic migraine or by comparing subjects that suffer from episodic and chronic migraine, is an indication that the subject suffers from chronic migraine, and c) wherein the identification of a higher expression level, as compared with a pre-established threshold value determined in subjects that suffer from chronic migraine or by comparing subjects that suffer from episodic and chronic migraine, is an indication that the subject suffers from episodic migraine.

In a preferred embodiment, the *in vitro* method further comprises: a) assessing the area of the right superior frontal gyrus by MRI of the subject, b) wherein a lower surface area of the right superior frontal gyrus, as compared with a pre-established threshold value determined in subjects that suffer from episodic migraine or by comparing subjects that suffer from episodic and chronic migraine, is an indication that the subject suffers from chronic migraine, and c) wherein a higher surface area of the right superior frontal gyrus, as compared with a pre-established threshold value determined in subjects that suffer from chronic migraine or by comparing subjects that suffer from episodic and chronic migraine, is an indication that the subject suffers from episodic migraine.

In a preferred embodiment, the biological sample is selected from serum-derived extracellular vesicles, plasma-derived extracellular vesicles, blood-derived extracellular vesicles, blood, serum or plasma, preferably wherein the biological sample is serum-derived extracellular vesicles.

The third aspect of the invention refers to the *in vitro* use of the expression level of miR-375 for the diagnosis of migraine, or for the differential diagnosis of chronic and episodic migraine.

It also refers to the *in vitro* use of the expression level of miR-375 in combination with the expression level of a miRNA selected from: miR-200a-3p, miR-141-3p, miR-454-3p and/or miR-16-2-3p for the diagnosis of migraine, or for the differential diagnosis of chronic and episodic migraine.

It also refers to the *in vitro* use of the expression level of the following combinations of miRNA: miR-375 and miR-200a-3p, miR-375 and miR-454-3p, and/or miR-375 and miR-16-2-3p for the diagnosis of migraine, or for the differential diagnosis of chronic and episodic migraine.

In a preferred embodiment, the *in vitro* use also involves the assessment the area of the right superior frontal gyrus by MRI.

In a preferred embodiment, the diagnosis of migraine is the diagnosis of chronic or episodic migraine.

The present invention also refers to:
- an *in vitro* method for the diagnosis of migraine which comprises: (a) assessing the expression level of miR-375, miR-200a-3p, miR-141-3p, miR-454-3p or miR-16-2-3p in a biological sample obtained from a subject, b) wherein the identification of a lower expression level, as compared with a pre-established threshold value determined in subjects that do not suffer from migraine, is an indication that the subject suffers from migraine.
- an *in vitro* method for the diagnosis of migraine which comprises: (a) assessing the expression level of two to five miRNAs selected from: miR-375, miR-200a-3p, miR-141-3p, miR-454-3p or miR-16-2-3p in a biological sample obtained from a subject, b) wherein the identification of a lower expression level, as compared with a pre-established threshold value determined in subjects that do not suffer from migraine, is an indication that the subject suffers from migraine.
- In a preferred embodiment, the *in vitro* method further comprises assessing the area of the right superior frontal gyrus by MRI wherein a lower surface area of the right superior frontal gyrus, as compared with a pre-established threshold value determined in subjects that do not suffer from migraine, is an indication that the subject suffers from migraine.
- In a preferred embodiment, the migraine is chronic migraine or episodic migraine.
- In a preferred embodiment, the biological sample is selected from serum-derived extracellular vesicles, plasma-derived extracellular vesicles, blood-derived extracellular vesicles, blood, serum or plasma.

In addition, the present invention also refers to:
- A computer implemented method, wherein a processing unit (hardware) and a software are configured to: a) receive any of the combinations of parameters of the above-mentioned methods (the expression level of miR-375, miR-200a-3p, miR-141-3p, miR-454-3p or miR-16-2-3p, and/or the area of the right superior frontal gyrus, preferably obtained by MRI), b) process these values in search of substantial variations or deviations, and c) provide an output through a terminal display of the variation or deviation of these values, wherein a variation or deviation of these values indicates that the subject suffers from migraine, episodic or chronic migraine.
- An *in vitro* method for identifying biomarker signatures for the diagnosis of migraine, which comprises assessing the expression level of miR-375, miR-200a-3p, miR-141-3p, miR-454-3p or miR-16-2-3p, and/or the area of the right superior frontal gyrus, preferably obtained by MRI, in a biological sample obtained from a subject, wherein a higher level, as compared with a pre-established threshold value determined in subjects who are not suffering from migraine, is an indication that the signature suitable for the diagnosis of migraine.
- In a preferred embodiment, the migraine is chronic migraine or episodic migraine.
- In a preferred embodiment, the biological sample is selected from serum-derived extracellular vesicles, plasma-derived extracellular vesicles, blood-derived extracelluluar vesicles, blood, serum or plasma.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of'. Thus, the phrase "consisting of" indicates that the listed elements are required or mandatory, and that no other elements may be present.
- The term "episodic migraine", according to The International Classification of Headache Disorders (3rd Edition, 2018), refers to a condition present in subjects that suffer from at least five attacks of migraine lasting 4-72 hours during their lifetime and who do not fulfil the criteria for chronic migraine (CM).
- The term "chronic migraine", according to The International Classification of Headache Disorders (3rd Edition, 2018), refers to a condition present in subjects that suffer from 15 or more migraine attacks per month, wherein at least eight of them present migraine features.
- The term "pre-established threshold value" refers to a value that is determined in subjects that belong to a reference group, or by comparing subjects that belong to two different groups. This value enables the discrimination between case and control subjects. The "pre-established threshold value" can be determined experimentally, empirically or theoretically. A "threshold value" can also be arbitrarily selected based on existing experimental and/or clinical conditions, as would be recognized by a person skilled in the art. The "threshold value" must be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.
- The term "extracellular vesicle" (EV) refers to small membrane-bound structures released by cells into the extracellular environment. They play essential roles in intercellular communication by transporting bioactive molecules such as proteins, lipids, nucleic acids, and metabolites between cells. EVs are classified into several types based on their biogenesis, size, and composition. The main types of extracellular vesicles include exosomes which are small EVs (typically 30-150 nm in diameter) formed through the inward budding of the endosomal membrane to generate multivesicular bodies (MVBs), which then fuse with the plasma membrane, releasing exosomes into the extracellular space; and microvesicles, also known as ectosomes, shedding vesicles, or microparticles, are larger than exosomes, ranging from 100 nm to 1 µm in diameter. They are formed by outward budding and fission of the plasma membrane. These different types of extracellular vesicles vary in size, biogenesis, cargo composition, and biological functions, but they collectively contribute to various physiological and pathological processes in multicellular organisms.

### Description of the figures

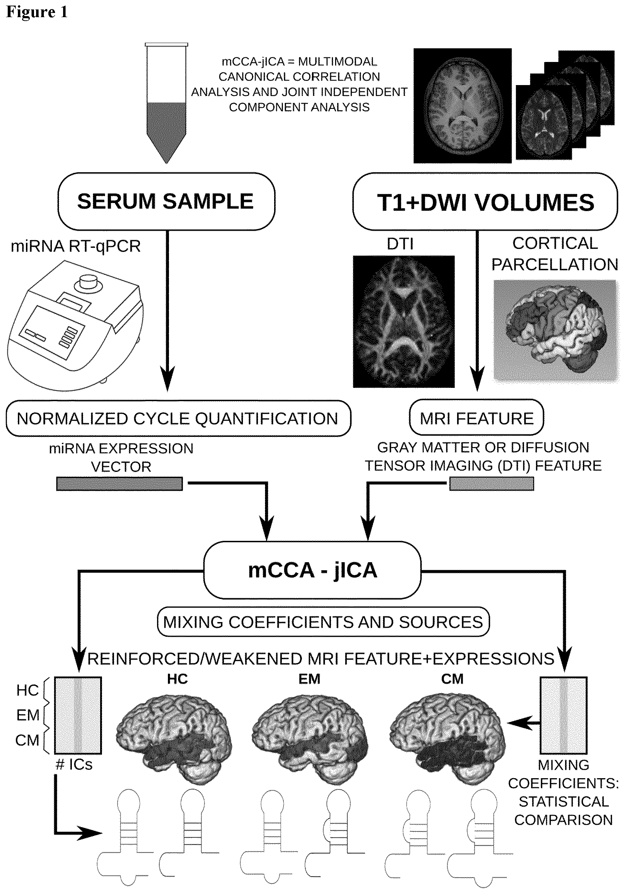

**Figure 1****.** Graphical representation of the joint assessment of miRNA and MRI features. The miRNA and MRI features were jointly analyzed using the multimodal canonical correlation analysis (mCCA) and joint Independent Component Analysis (jICA) methodology (Planchuelo-Gómez et al. 2021). The MRI features were gray matter morphometry and diffusion tensor imaging descriptors.

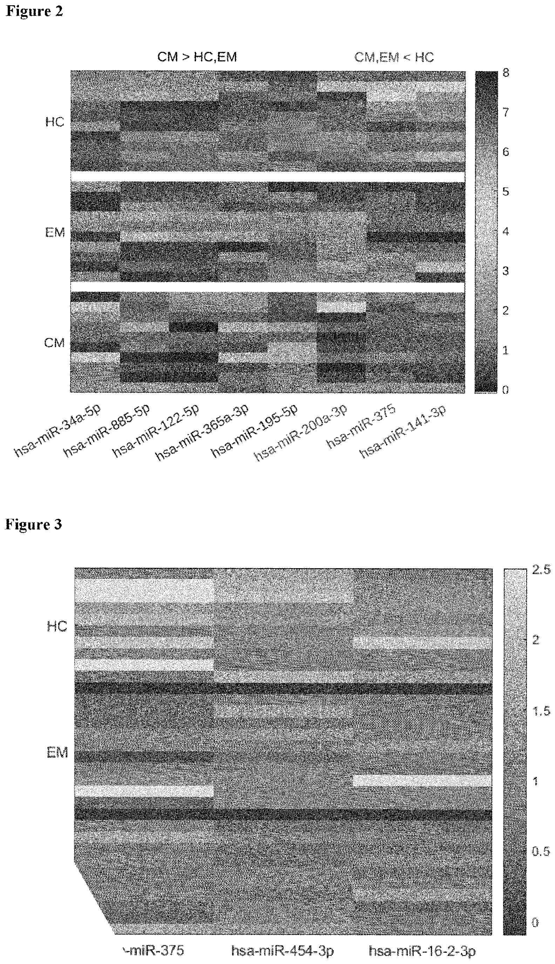

**Figure 2****.** Heatmap of the differentially expressed miRNAs following the two main identified patterns identified by joint assessment of miRNA and MRI features. The first five miRNAs were over-expressed in patients with CM compared to patients with EM and HC, and the last three miRNAs were under-expressed in CM and EM patients compared to HC. Each colored row represents a subject.
**Figure 3****. Heatmap of the top three differentially expressed serum extracellular vesicles miRNA in HC, EM and CM.** miRNA expression values are Relative Quantities (RQ) (n=30). Each colored row represents a subject. The top three differentially expressed exosome miRNA levels were miR-375, miR-454-3p and miR-16-2-3p, which were lower in patients with migraine compared to healthy controls (p<0.05) in the ANOVA or Kruskal-Wallis test.

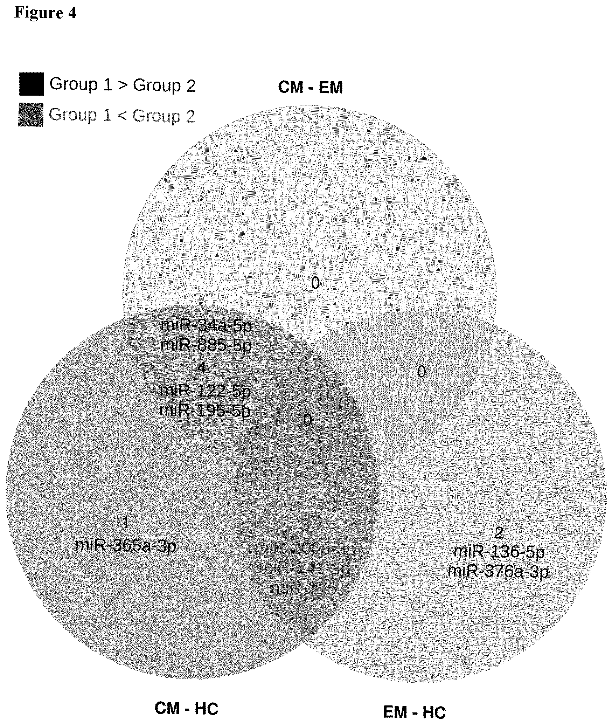

**Figure 4****.** Venn's diagram showing the diverse patterns of the miRNA changes between groups in association with MRI-based brain structural changes. The miRNAs in black reflect higher values in CM compared to EM or HC, or in EM compared to HC, while the miRNAs in red reflect lower miRNA expression in CM and EM compared to HC.

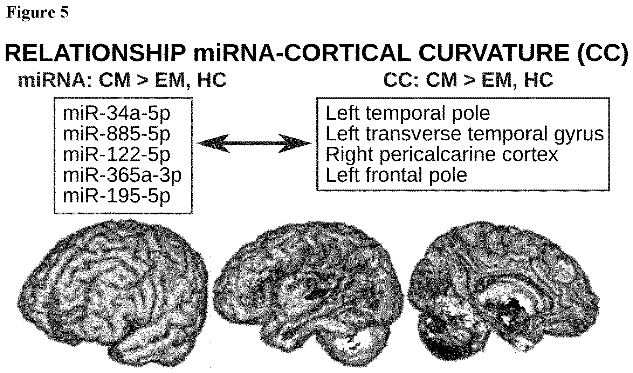

**Figure 5****.** Main miRNAs and gray matter regions involved in changes in CM compared to EM and HC of miRNA expression and cortical curvature.

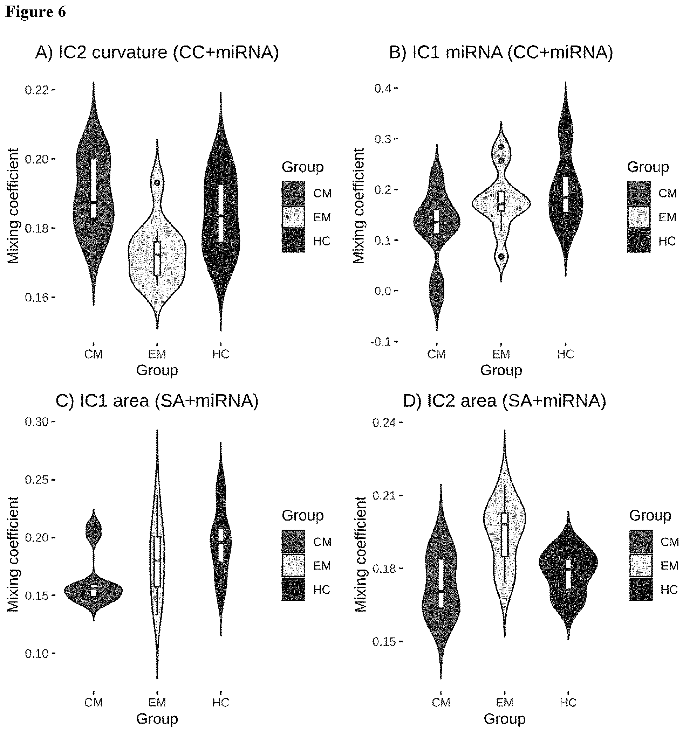

**Figure 6****.** Violin plots of the mixing coefficients that showed statistically significant differences in the mCCA-jICA analysis. A) Second cortical curvature (CC) component of the joint analysis of CC and micro-RNA (miRNA). Coefficients showed higher values in CM compared to EM. B) First miRNA component of the joint analysis of CC and miRNA. Coefficients showed lower values in CM compared to HC. C) First surface area (SA) component of the joint analysis of SA and miRNA. Coefficients showed lower values in CM compared to HC. D) Second SA component of the joint analysis of SA and miRNA. Coefficients showed higher values in EM compared to HC, and lower values in CM compared to EM.

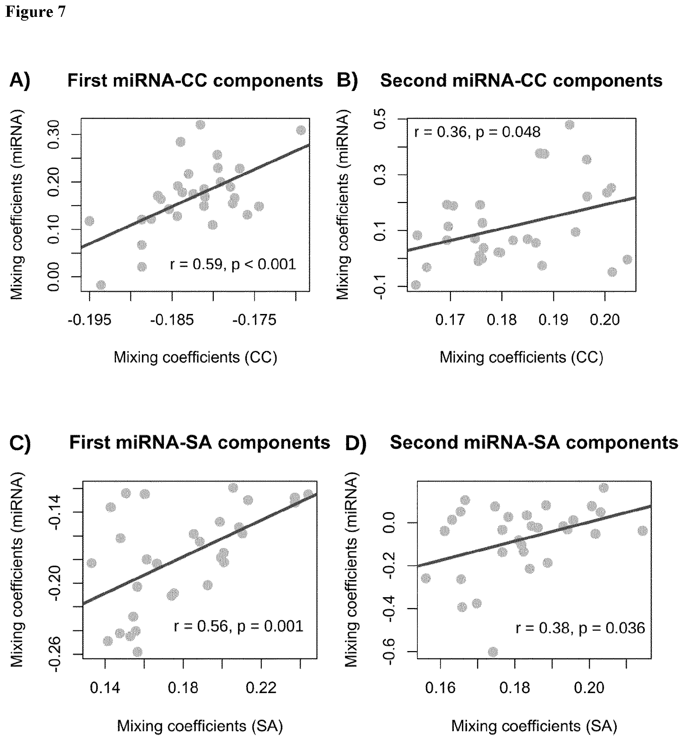

**Figure 7****.** Scatter plots of the joint analysis of cortical curvature (CC) or surface area (SA) and micro-RNA (miRNA). Each plot shows the Pearson's correlation between at least one mCCA-jICA component with statistically significant differences between clinical groups.

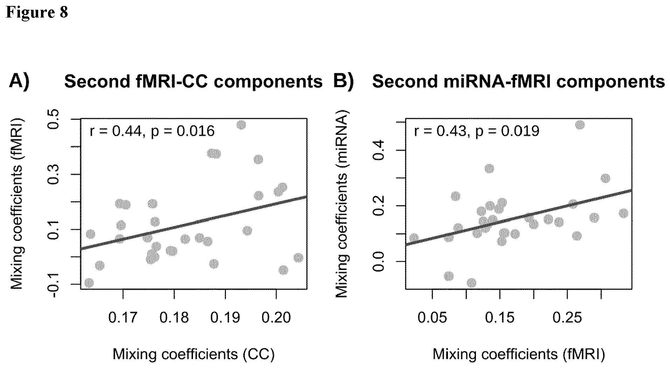

**Figure 8****.** Scatter plots of the joint analysis of cortical curvature (CC) or micro-RNA (miRNA) with functional MRI. Each plot shows the Pearson's correlation between at least one mCCA-jICA component with statistically significant differences between clinical groups.

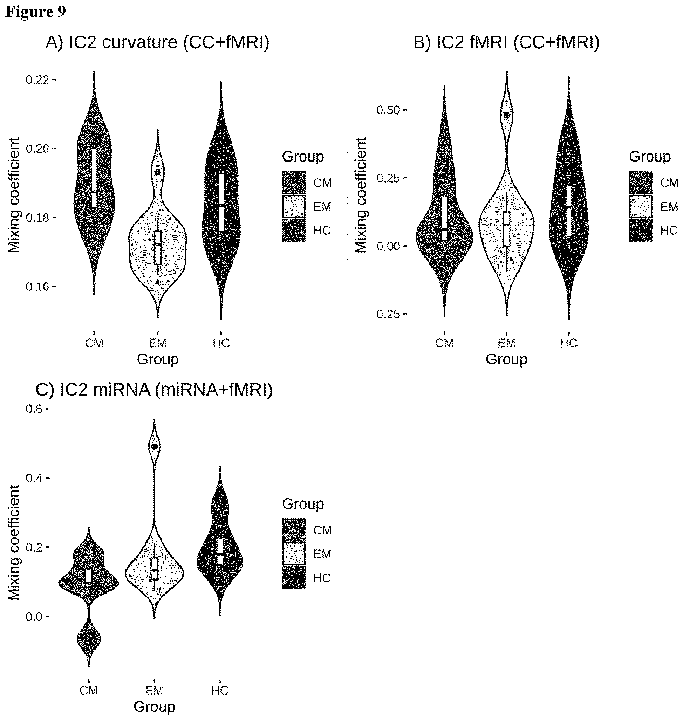

**Figure 9****.** Violin plots of the mixing coefficients that showed statistically significant differences in the mCCA-jICA analysis. A) Second cortical curvature (CC) component of the joint analysis of CC and functional MRI (fMRI). Coefficients showed higher values in CM compared to EM. B) Second fMRI component of the joint analysis of CC and fMRI. Coefficients showed higher values in CM compared to EM and HC. C) Second micro-RNA (miRNA) component of the joint analysis of fMRI and miRNA. Coefficients showed lower values in CM compared to HC.

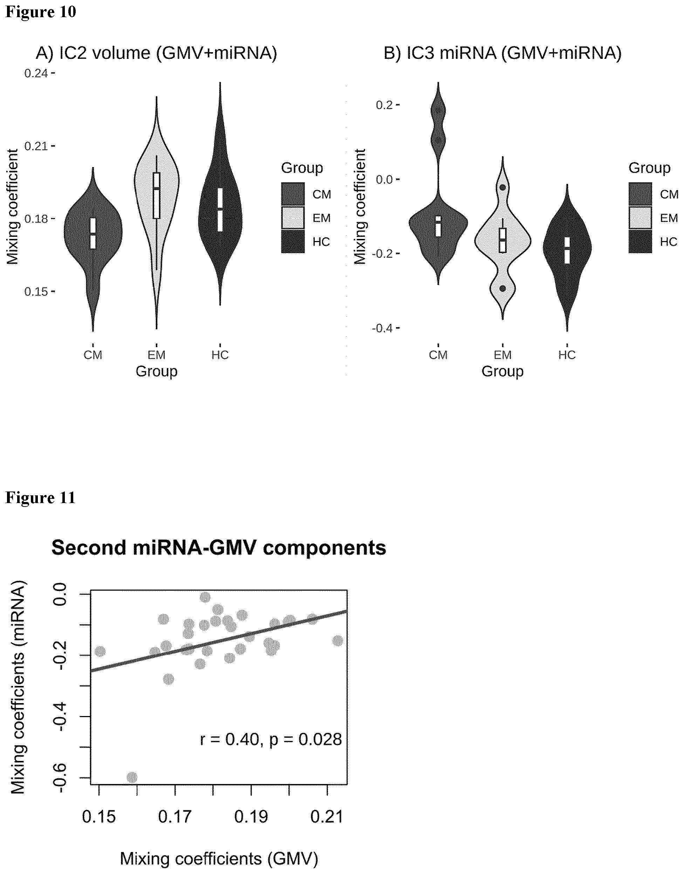

**Figure 10****.** Violin plots of the mixing coefficients that showed statistically significant differences in the mCCA-jICA analysis of the joint assessment of gray matter volume and micro-RNA (miRNA). A) Second volume component of the joint analysis of volume and miRNA. Coefficients showed lower values in CM compared to EM. B) Third component of the joint analysis of gray matter volume and miRNA. Coefficients showed higher values in CM compared to HC.
**Figure 11****.** Scatter plot of the joint analysis of gray matter volume (GMV) with micro-RNA (miRNA). The plot shows the Pearson's correlation between the mCCA-jICA second components.

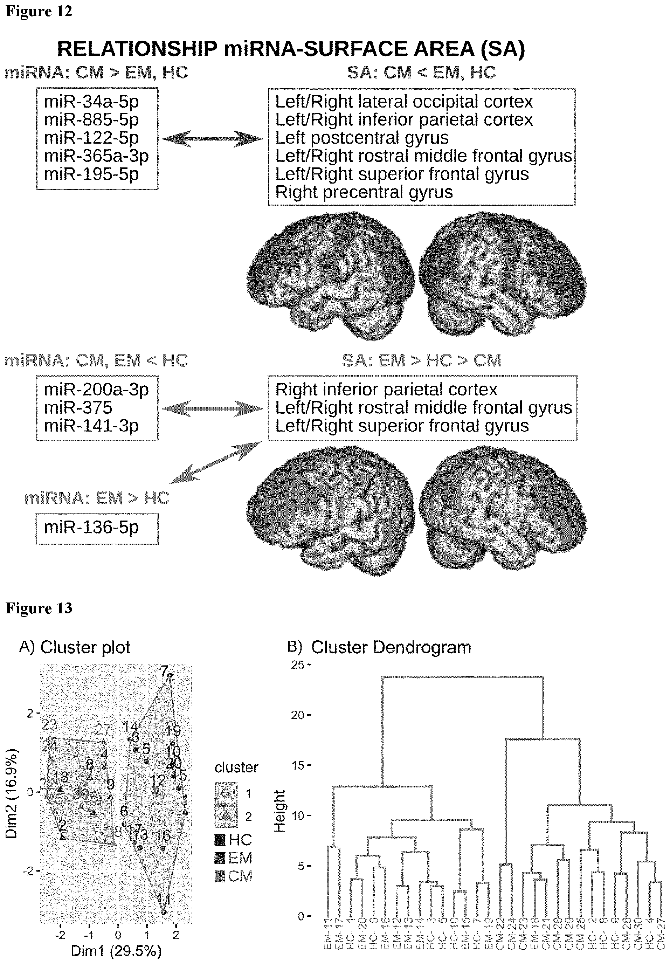

**Figure 12****.** Main miRNAs and gray matter regions involved in changes between CM, EM and HC of miRNA expression and surface area.
**Figure 13****.** Clusterization of the HC, EM and CM groups based on cortical curvature, surface area, gray matter volume and miRNA expression that showed differences following the mCCA-jICA assessment via hierarchical clustering. A) Cluster plot. B) Cluster dendrogram.

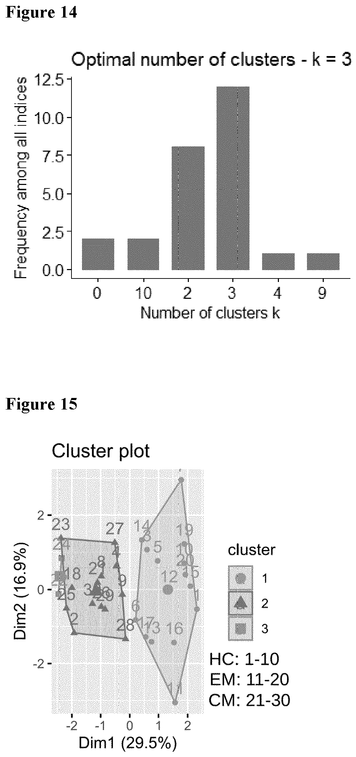

**Figure 14****.** Optimal number of clusters from the principal components of the gray matter morphometry features and micro-RNA (miRNA). The optimal number of clusters for most methods was 3, followed by 2. The numbers between 0 and 10 not included in the figure were not considered by any technique.
**Figure 15****.** Results from k-means clustering considering three clusters. It can be appreciated that cluster 3 is just composed of two patients with chronic migraine (CM) and are considered outliers. Subjects 1-10 are healthy controls (HC), 11-20 are episodic migraine (EM) patients, and 21-30 CM patients. One cluster included six HCs and nine EM patients (cluster 1), and another one (cluster 2) included four HCs, one EM patients and eight CM patients.

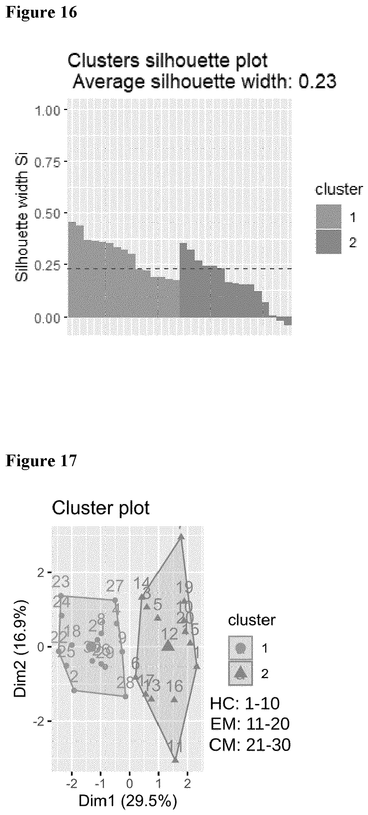

**Figure 16****.** Silhouette profile of the hierarchical clustering considering two clusters.
**Figure 17****.** Results from k-means clustering considering two clusters. Subjects 1-10 are healthy controls (HC), 11-20 are episodic migraine (EM) patients, and 21-30 chronic migraine (CM) patients. One cluster included six HCs and nine EM patients (cluster 1), and the other one (cluster 2) included four HCs, one EM patients and 10 CM patients.

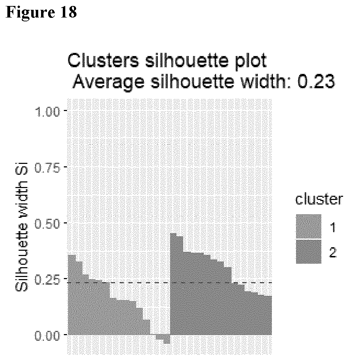

**Figure 18****.** Silhouette profile of the k-means clustering considering two clusters.

### Detailed description of the invention

The present invention is illustrated by means of the Examples set below without the intention of limiting its scope of protection.

### Example 1. Material and Methods

### Example 1.1. Participants and protocol approval

The study was approved by the Institutional Review Board of Hospital Universitario de Valladolid, Spain. All participants provided written informed consent before participation. We recruited patients treated for migraine in the Headache Outpatient Clinic. Patients had a diagnosis of migraine with or without aura, confirmed by a neurologist specialized in headache, according to the International Classification of Headache Disorders, 3rd edition (ICHD-3) («Headache Classification Committee of the International Headache Society (IHS) The International Classification of Headache Disorders, 3rd Edition», 2018). HC were recruited among hospital staff and non-related acquaintances of patients who had no past or first-degree familial history of any recurrent primary or secondary headache disorder. To diminish confounding factors, the inclusion criteria for all groups were: female sex, 18-45 years old (non-menopausal) with no preventive medication. Participants were included from January 2022 to September 2022.

### Example 1.2. Blood samples

Blood samples were collected in glass red-top serum vacutainer tubes without additives (BD, Franklin Lakes, NJ); and kept at room temperature for 45 minutes before centrifugation at 1800 rpm for 10 minutes to separate serum, and then stored at -80°C until use. Serum was frozen within 2 hours of the blood draw.

### Example 1.3. Purification of extracellular vesicles (EV) using polyethylene glycol (PEG)

PEG EV precipitation was performed according to the protocol (Andreu Z. 2016). Briefly, 200 ***µ***l of serum was mixed with 100 ***µ***l of PEG 6000 (Merck) solution (50% PEG 6000 in 375 mM NaCl). Serum samples were mixed with PEG solution by repeated pipetting and incubated form 30 minutes in ice. After incubation, samples were centrifuged at 1500g for 30 minutes at 4°C. Supernatant was discarded by aspiration to eliminate any residual PEG solution. Pellet was then resuspended in 200 ***µ***L of H20 for RNA extraction and frozen at -20°C.

### Example 1.4. miRNA extraction

miRNA EV was extracted according to the Maxwell^{®} RSC miRNA Plasma and Serum Kit Technical Manual manufacturer instructions (AS 1680). Each resuspended sample (200 ***µ***L) was mixed with 80 ***µ***L of proteinase K, 230 ***µ***L of lysis buffer and 1 ***µ***L of spike-in per sample, followed by 5 seconds vortex, and incubation at 37° for 15 minutes. DNAse I solution and sample were added to the Maxwell cartridge and processed for 70 minutes.

### Example 1.5. Reverse Transcription (RT) and RT-qPCR Assays

The isolated RNA was reverse transcribed to complementary DNA (cDNA) using miRCURY LNA RT Kit (Qiagen) following manufacturer's instructions and quality control of this process was carried out using cel-miR-39 and Unisp6 RNA spike-in templates. Prepared complementary DNAs were stored at -20 °C until use.

The miRNAs were measured by qPCR using miRCURY LNA miRNA Focus PCR Panels (QIAGEN), which comprises 179 commonly found miRNAs in human serum, using miRCURY LNA SYBR^{®}Green PCR Kit (QIAGEN). QuantStudio 5 Real-Time PCR System (ThermoFisher Scientific) was used for qPCR plates reading. A positive signal was defined by a threshold cycle (Ct) cut-off value of < 36 cycles in the PCR run.

### Example 1.6. MRI acquisition

High resolution 3D T1-weighted volumes were acquired using a Philips Achieva 3 T MRI unit (Philips Healthcare, Best, The Netherlands) with a 32-channel head coil for the extraction of the gray matter morphometry parameters. To acquire diffusion descriptors to analyze the white matter structure, diffusion-weighted MRI data were acquired. The information about functional changes was obtained with resting-state functional MRI (fMRI) (Smith et al., 2004). For the detection of white matter hyperintensities, T2-weighted and fluid attenuated inversion recovery (FLAIR) sequences were additionally obtained. All the MRI volumes were acquired in the same session.

The acquisition parameters of the T1-weighted volumes were the following: Turbo Field Echo sequence, repetition time (TR) = 8.1 ms, echo time (TE) = 3.7 ms, flip angle = 8°, spatial resolution of 1 × 1 × 1 mm3 in a 256 × 256 matrix size covering the whole brain in 170 sagittal slices.

Next, the diffusion MRI data were obtained. The acquisition parameters were TR = 9000 ms, TE = 86 ms, flip angle = 90°, two non-diffusion weighted volumes with opposite phase encoding direction, 61 diffusion-weighted volumes with b = 1000 s/mm2, and spatial resolution of 2 × 2 × 2 mm3 in a 128 × 128 matrix size covering the whole brain in 66 axial slices.

The acquisition parameters of the resting-state fMRI volumes were TR = 3000 ms, TE = 30 ms, flip angle = 80°, spatial resolution of 3 × 3 × 4 mm3 in an 80 × 80 matrix size covering the whole brain in 35 axial slices. There was a total of 197 repetitions and the subjects were instructed to close their eyes without sleeping during the fMRI acquisition.

The T2-weighted data were acquired using a Turbo Spin Echo sequence, TR = 3000 ms, TE = 80 ms, flip angle = 90°, spatial resolution of 0.43 × 0.43 × 5 mm3 in a 560 × 560 matrix size covering the whole brain in 28 axial slices.

For the FLAIR sequence, high resolution 3D volumes were acquired with TR = 4800 ms, TE = 308 ms, inversion time (TI) = 1650 ms, flip angle = 90°, spatial resolution of a 0.56 × 1.04 × 1.04 mm3 in a 560 × 560 matrix size covering the whole brain in 321 sagittal slices.

### Example 1.7. Extraction of features

The assessed features were the miRNA microarray expression profile, four gray matter morphometry parameters, and four diffusion tensor imaging (DTI) descriptors. The gray matter morphometry parameters were cortical curvature and thickness, cortical surface area, and gray matter volume. The cortical curvature reflects the folding of the cortex, the cortical thickness the distance between the pial and the surface in contact with the white matter, and the surface area is the area occupied by the surface that separates the white and the gray matter. The gray matter volume is related to the cortical thickness and surface area. The DTI parameters were fractional anisotropy (FA), axial diffusivity (AD), radial diffusivity (RD) and mean diffusivity (MD). The FA has been related to the integrity of white matter in association with the degree of directionality of water diffusivity, the AD has been associated with the diffusion in the main direction of the white matter fibers, and the RD and MD with the diffusion in the perpendicular direction and global measure of water diffusion, respectively. The functional feature from fMRI is based on the mathematical correlation of the signal through time between diverse gray matter regions of interest (ROIs).

### Example 1.8. qPCR Data Analysis for miRNA expression

A quality control of the RNA extraction was performed by means of the spike-in previously added to the extraction. Ct values above 36 were discarded for the global mean calculation. Interplate calibration (IPC) was performed using UniSp3 as described in the kit's Handbook. Then, Relative Quantities (RQ) were calculated as the log base 2 values of the Ct difference between miRNAs and Unisp2 RNA spike-in as previously described (20). Normalization factor (NF) was calculated as the geometric mean of the RQs of all expressed targets per sample. Normalized Relative Quantities (NRQ) were obtained by dividing the RQs by the sample specific NF.

### Example 1.9. Gray matter morphometry

The T1-weighted images were processed with the FreeSurfer parcellation pipeline (v6.0.0) to extract the four gray matter morphometry parameters. All the parameters were obtained for 68 cortical ROIs from both hemispheres and the gray matter volume was additionally extracted for the cerebellum and 14 bilateral subcortical regions, for a total of 84 regions from the atlas Desikan-Killiany (Desikan et al., 2006). Briefly, the main steps of the FreeSurfer pipeline were skull stripping, segmentation of gray and white matter, normalization, tessellation of the gray-white matter boundary, and surface deformation.

### Example 1.10. Diffusion descriptors

The diffusion-weighted data were firstly preprocessed. The initial preprocessing steps were denoising and Gibbs ringing removal (Cordero-Grande et al., 2019; Kellner et al., 2016; Veraart et al., 2016). Then, the images were corrected for eddy currents, motion, and B0 and B1 field inhomogeneities (Andersson & Sotiropoulos, 2016). The whole preprocessing pipeline was conducted using the MRtrix software (Tournier et al., 2019).

A brain mask was obtained from the preprocessed data, and the four DTI parameters were obtained from the masked volumes after extracting the diffusion tensor at each voxel using an ordinary least squares estimation. After the four DTI-based maps were estimated, the FA images were non-linearly registered to a template of the averaged FA volumes (FMRIB-58) in the Montreal Neurological Institute (MNI) space. A mean FA image was generated and thinned to create a mean FA skeleton of the white matter tracts, using a value of 0.2 as threshold to distinguish white from gray matter. Then, the FA map from each subject was projected onto the white matter skeleton, and this process was repeated for the remaining DTI parameters using the same registration operations as those employed for the FA. Finally, the parameters were obtained for the skeletonized white matter regions of interest using the Johns Hopkins University ICBM-DTI-81 White-Matter Labels Atlas (Oishi et al., 2008) extracting the mean value for each of the 48 regions.

### Example 1.11. FMRI analysis

As in the extraction of the diffusion features, firstly a series of pre-processing steps were applied to the fMRI data. These steps were motion correction, outlier detection, co-registration to the T1-weighted data for each subject, structural segmentation and denoising with a bandpass filter with 0.01 Hz and 0.1 Hz as cut-off frequencies (Zhang et al., 2001).

The functional feature was the Pearson's correlation value of the blood-oxygen-level-dependent (BOLD) signal provided by the fMRI data between diverse gray matter ROIs. The ROIs were the same as in the gray matter morphometry analysis previously described. To avoid the limitations of the correlation values, with a range between -1 and 1, the Fisher r-to-Z transformation was applied to the raw correlation values and the functional feature was expressed as a Z-score.

### Example 1.12. Joint assessment of the features

The miRNA and gray matter morphometry features were jointly analyzed using the multimodal canonical correlation analysis (mCCA) and joint Independent Component Analysis (jICA) methodology described in (Planchuelo-Gómez et al., 2021). Briefly, the combination of both methods allows the extraction of the independent subcomponents that compose each set of features (miRNA and one MRI feature in this study) that present maximum correlation between the two modalities of interest. The procedure is summarized in **Figure 1****.**

To determine the number of components for the two types of features, the Horn's method was employed (Horn, 1965). Following this approach, the eigenvalues for each set of features and from randomly generated data with the same size were obtained, with 500 repetitions for the random datasets. The eigenvalues from the original data were compared against the 95th percentile value from the 500 random repetitions. The number of retained components per modality was the number of eigenvalues from the original features that preserved higher values than the 95th percentile from the corresponding eigenvalue from the random data. If both modalities presented a different number of retained components, the highest value was taken for the joint analysis.

For the secondary analysis, the procedure was repeated between the features with significant differences and the fMRI features. The number of components was preserved with respect to the original assessment of miRNA and structural MRI features, and the components were obtained for the fMRI data. The results of the functional features were focused regions that were highlighted for the joint miRNA-structural MRI analysis, i.e., no connections without identified ROIs in the main analysis were considered.

### Example 1.13. Statistical analysis

There were four pairs of features that were assessed, considering the close relationship between three of the four morphometry parameters. Each morphometry parameter was jointly assessed with the miRNA data.

After the mCCA-jICA procedure, each independent component is quantified by the mixing coefficients, expressed as Z-scores. The comparison of the mixing coefficients between the three clinical groups determines whether the identified differences for each modality are correlated or independent. Specific details are described in (Planchuelo-Gómez et al., 2021).

The mixing coefficients and the demographic and clinical quantitative variables were firstly assessed for normality with the Shapiro-Wilk test. To test the equality of variance hypothesis between the clinical groups, the Levene's test was employed. If all hypotheses were met, the analysis of variance (ANOVA) test was used, and the Kruskal-Wallis test otherwise. Two-by-two post hoc comparisons were conducted with the Tukey-Kramer or Conover-Iman tests for the ANOVA and Kruskal-Wallis tests, respectively. For the comparison of clinical variables between the two groups of migraine patients, the t-test for equal or distinct variances was employed for normal variables, and the Mann-Whitney's U test otherwise.

For the components with statistically significant differences, to determine the specific miRNA and/or regions with higher weight on the mixing coefficients of the independent components, an automatic detection of outliers was conducted using the procedure available in MATLAB software (R2022b release). Positive and negative outliers were considered, representing the outliers higher or lower expression in a particular clinical group. The outliers were extracted for the components with mixing coefficients for the modality that showed statistically significant differences, considering the three groups. The outliers were also assessed for the corresponding component of the other modality if the mixing coefficients showed significant correlation with a modality that showed significant differences.

Two sets of correlations assessments were conducted. For each pair of mixing coefficients from the independent components of the miRNA and gray matter morphometry features, the correlation analysis was performed to determine whether the changes in both modalities were correlated. The Pearson's correlation coefficient was extracted.

Finally, a multimodal signature was assessed based on the area under the curve (AUC) performance, obtained from logistic regression, of the comparisons between HC, EM and CM patients regarding migraine diagnosis and differentiation between EM and CM. The candidate miRNAs and morphometry parameters for this assessment were those with differences between migraine groups and HC identified through the mCCA-jICA multimodal technique. Additionally, miRNAs with statistically significant differences between at least one migraine group and HC, following the same statistical analysis as for the comparisons of the mixing coefficients and the demographic and clinical variables, were considered.

For all comparisons, the level of statistical significance was set at p < 0.05.

### Example 1.14. Machine Learning classification

In addition to the mCCA-jICA assessment, a further unsupervised machine learning task was conducted to classify the HC and the patients with EM and CM. The objective of this analysis was the classification of the subjects, without considering the established clinical groups. The data employed to carry out the classification were obtained from a Principal Component Analysis (PCA) of the specific features differentially expressed according to the mCCA-jICA technique. Per feature, i.e., miRNA and each morphometry parameter, two principal components were extracted from the original data, selecting the regions or miRNA determined as outliers in the mCCA-jICA analysis. The principal components were expressed as Z-scores to avoid bias from the parameters with the highest values.

Once the principal components were obtained for all modalities after the mCCA-jICA analysis, the optimal number of clusters of the unsupervised learning task was determined using 26 different indexes. Multiple values were used to prevent from the bias that may be caused from using an exclusive criterion or a small number of indexes. From the 26 values for the optimal number of clusters, the final value was obtained using the majority rule, i.e., the value obtained for a higher number of indexes. In case of a value extracted the same number of times for different criteria, the lowest number of clusters was chosen. In case of detecting a cluster with a few outliers, the analysis was repeated reducing one cluster per group of outliers. The procedure was implemented with the NbClust package included in R statistical software (v4.1.2), with further details available at (Charrad et al., 2014).

After selecting the number of clusters, the k-means and Ward's hierarchical clustering algorithms were employed to obtain the final clusters. Two methods were employed to check that results were independent from a specific clustering algorithm. A total of different 50 random centroids or seeds were used to initialize the techniques and those with the best silhouette profile were finally established for the final extraction of the clusters (Rousseeuw, 1987).

### Example 2. Results

A total of 30 patients (10 HC, 10 patients with EM and 10 patients with CM) were analyzed. Regarding the demographic and clinical characteristics, statistically significant differences between the groups were only appreciated for headache and migraine frequency, as expected.

**Table 1** shows the demographic and clinical characteristic of the sample.

**Table 1. Clinical and demographic characteristic of the sample.**

| | HC (n=10) | EM (n=10) | CM (n=10) | Statistical Test |
|---|---|---|---|---|
| Age (years) | 26.1 ± 3.1 | 33.1 ± 9.0 | 33.4 ± 9.8 | χ²_{(2, N=30)} = 3.80, p = 0.15^{a} |
| Sex (female/male) | 10/0 | 10/0 | 10/0 | NA |
| Frequency of headache (days/month) | NA | 3.8 ± 2.2 | 22.2 ± 4.2 | t₍₁₈₎ = -12.4, p < 0.001^{b} |
| Frequency of migraine (days/month) | NA | 3.8 ± 2.2 | 11.4 ± 4.7 | U = 98, p < 0.001^{c} |
| Duration of migraine (years) | NA | 17.0 ± 9.7 | 16.8 ± 8.2 | t₍₁₈₎ = 0.05, p = 0.96^{b} |
| Duration of CM (months) | NA | NA | 32.7 ± 33.3 | NA |
| Medication overuse, N (%) | NA | 0 (0%) | 6 (60%) | NA |

| | | | | |
|---|---|---|---|---|
| Quantitative variables are expressed as mean ± standard deviation. NA = not available. a = Kruskal-Wallis test, b = t-test (equal variances), c = Mann-Whitney's U. | | | | |

### Example 2.1. Joint analysis of miRNA and gray matter morphometry

For all assessments, a total of four components were obtained per modality, i.e., four miRNA and four gray matter morphometry components or DTI-based parameters for each pair. Regarding the gray matter morphometry parameters, statistically significant differences between the three groups were found for all pairs except the assessment with miRNA and cortical thickness. For the diffusion descriptors, no statistically significant differences between the groups were obtained in relation to miRNA.

Considering all the mCCA-jICA assessments, three main patterns were identified in the miRNA analysis (**Figure 2**). The first one was higher miRNA expression in CM patients compared to HC, and the second one was the opposite, i.e., lower miRNA expression in CM compared to HC. For some miRNA in these patterns, additionally, higher and lower miRNA expression in EM compared to HC, and in CM compared to EM, were appreciated in the first (CM > EM > HC) and second (CM < EM < HC) patterns, respectively. The third pattern was less frequent than the other two and showed higher miRNA expression values in EM compared to HC.

The main miRNAs of the first pattern (CM > EM, HC) were miR-34a-5p, miR-885-5p, miR-122-5p, miR-365a-3p, and miR-195-5p. For the second pattern (CM, EM < HC), the main miRNAs were miR-200a-3p, miR-375 and miR-141-3p. The heatmap of the values for all subjects for the mentioned miRNA is shown in **Figure 2****.**

Regarding the third pattern (EM > HC), the main miRNAs were miR-136-5p and miR-376a-3p. The summary of the three patterns with the pairwise comparisons is shown in **Figure 4****.**

In the following subsections, the results for each pair of miRNA and gray matter morphometry parameters with statistically significant differences for at least a pair of groups are described.

### Example 2.2. MiRNA and cortical curvature

The mixing coefficients of two components presented statistically significant differences between the clinical groups. The first miRNA component showed lower mixing coefficient values in CM compared to HC (pANOVA = 0.047, pTukey = 0.043), including specific miRNA that followed the three patterns previously described. Accordingly, the first curvature component outliers showed higher curvature values in CM compared to HC, and lower values in the left entorhinal cortex. The second curvature component showed higher mixing coefficient values in CM compared to EM (pANOVA = 0.004, pTukey = 0.003). In line with this result, the second miRNA component showed higher expression in CM compared to EM. The first and second components for the two modalities showed statistically significant correlation. The correlation values were r = 0.59 (p < 0.001) for the first components, and r = 0.36 (p = 0.048) for the second components. The main miRNAs and regions involved in the correlated changes of miRNA expression and cortical curvature are shown in **Figure 5****.** The specific miRNAs and regions that were classified as outliers, together with the distribution and correlation analysis of the mixing coefficients with statistically significant differences, are shown in **Tables 2-5** and **Figures 6-7****.**

**Table 2. Cortical gray matter regions highlighted as outliers in the first component of cortical curvature of the mCCA-jICA analysis of cortical curvature and miRNA.**

| ROI | CM < HC | EM < HC | CM > HC | EM > HC | CM > EM |
|---|---|---|---|---|---|
| L entorhinal cortex | X | X | | | |
| L rostral anterior cingulate gyrus | | | X | X | |
| R rostral anterior cingulate gyrus | | | X | X | |
| R entorhinal cortex | | | X | X | |
| L parahippocampal gyrus | | | | | X |
| L temporal pole | | | X | | X |
| R temporal pole | | | | | X |
| L transverse temporal gyrus | | | X | | X |
| R pars orbitalis | | | | | X |
| R pericalcarine cortex | | | X | | X |

| | | | | | |
|---|---|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | | | |

**Table 3. Cortical gray matter regions highlighted as outliers in the second component of cortical curvature of the mCCA-jICA analysis of cortical curvature and miRNA.**

| ROI | EM < HC | CM > HC | EM > HC | CM > EM |
|---|---|---|---|---|
| L banks of the superior temporal sulcus | | | | X |
| L parahippocampal gyrus | | X | | X |
| L frontal pole | X | | | X |
| R frontal pole | | | | X |
| L paracentral lobule | | X | X | |
| R entorhinal cortex | | X | X | |
| R precuneus cortex | | X | X | |

| | | | | |
|---|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | | |

**Table 4. Micro-RNAs (miRNAs) highlighted as outliers in the first component of miRNA of the mCCA-jICA analysis of cortical curvature and miRNA.**

| miRNA | CM < HC | EM < HC | CM < EM | CM > HC | EM > HC | CM > EM |
|---|---|---|---|---|---|---|
| miR-200a-3p | X | X | | | | |
| miR-141-3p | X | X | | | | |
| miR-485-3p | X | | X | | | |
| miR-375 | X | X | | | | |
| miR-200c-3p | X | X | | | | |
| miR-1 | X | X | | | | |
| miR-483-5p | X | X | X | | | |
| miR-497-5p | X | | | | | |
| miR-335-3p | X | X | | | | |
| miR-7-5p | X | | X | | | |
| miR-194-5p | | | | X | | X |
| miR-34a-5p | | | | X | | X |
| miR-193a-5p | | | | X | X | X |
| miR-885-5p | | | | X | | X |
| miR-99a-5p | | | | X | | X |
| miR-122-5p | | | | X | | X |
| miR-365a-3p | | | | X | | X |
| miR-126-5p | | | | X | | |
| miR-195-5p | | | | X | | X |
| miR-125b-5p | | | | X | | X |
| miR-376c-3p | | | | X | X | |
| miR-136-5p | | | | | X | |
| miR-376a-3p | | | | | X | |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | | | | |

**Table 5. Micro-RNAs (miRNAs) highlighted as outliers in the second component of miRNA of the mCCA-jICA analysis of cortical curvature and miRNA.**

| miRNA | CM < HC | EM < HC | CM < EM | CM > HC | EM > HC | CM > EM |
|---|---|---|---|---|---|---|
| miR-122-5p | X | X | | | | |
| miR-483-5p | X | X | | | | |
| miR-885-5p | | | | X | | X |
| miR-200a-3p | | | | X | | X |
| miR-409-3p | | | | X | | X |
| miR-485-3p | | | | X | | X |
| miR-let-7f-5p | | | | X | | |
| miR-874-3p | | | | X | | X |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, R = right. | | | | | | |

For the secondary analysis focused on the relationship between these results and the fMRI-based features, two distinct functional patterns were associated with the pattern shown in **Figure 5****.** The association of this pattern was found in the mCCA-jICA assessment of cortical curvature with fMRI (see **Tables 6-7** and **Figures 8-9** for details). On the one hand, CM patients presented lower functional connectivity in the connection between the left frontal pole and the right temporal pole. On the other hand, in CM, higher functional connectivity was identified between the left frontal pole and the right medial orbitofrontal gyrus.

**Table 6. Gray matter regions highlighted as outliers in the second component of cortical curvature of the mCCA-jICA analysis of cortical curvature and functional MRI (fMRI).**

| ROI | CM < HC | EM < HC | CM > HC | EM > HC | CM > EM |
|---|---|---|---|---|---|
| L entorhinal cortex | X | X | | | |
| R entorhinal cortex | | | X | X | |
| L banks of the superior temporal sulcus | | | | | X |
| L parahippocampal gyrus | | | X | | X |
| L frontal pole | | X | | | X |
| R frontal pole | | | | | X |
| R rostral anterior cingulate gyrus | | | X | X | |

| | | | | | |
|---|---|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | | | |

**Table 7. Connections between gray matter regions highlighted as outliers in the second component of functional MRI (fMRI) of the mCCA-jICA analysis of cortical curvature and fMRI.**

| ROIs | CM < HC | EM < HC | CM < EM | CM > HC | EM > HC | CM > EM |
|---|---|---|---|---|---|---|
| L frontal pole - R temporal pole | X | | X | | X | |
| L frontal pole - R medial | | X | | X | | X |
| orbitofrontal gyrus | | | | | | |
| L banks of the superior temporal sulcus - L middle temporal gyrus | X | X | | | | |
| R frontal pole - R hippocampus | | X | | | | |
| L lingual gyrus - R frontal pole | X | | X | | | |
| R fusiform gyrus - R frontal pole | X | | X | | | |
| R entorhinal cortex - R hippocampus | X | X | | | | |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | | | | |

### Example 2.3. MiRNA and surface area

The mixing coefficients of the first two surface area components showed statistically significant differences and presented significant correlation with the corresponding two miRNA components. The first area component presented the highest correlation with the corresponding miRNA component (r = 0.56, p = 0.001), and the mixing coefficient values were lower in CM compared to HC (pANOVA = 0.049, pTukey = 0.038). In relation to this pattern, the corresponding miRNA component outliers reflected a group of miRNAs with lower expression in CM compared to HC, and another one with the opposite result (higher values in CM). The second area component (r = 0.38, p = 0.036) showed higher values in EM compared to CM and HC (pANOVA < 0.001, pTukey = 0.007). Accordingly, a group of miRNAs of the second component following the outlier assessment showed higher values in EM compared to HC and CM. The main miRNAs and regions involved in the correlated changes of miRNA expression and cortical curvature are shown in **Figure 5****.** The specific miRNAs and regions that were classified as outliers, together with the distribution and correlation plots of the mixing coefficients with statistically significant differences, are shown in the **Tables 8-11** and **Figures 6-7****.**

**Table 8. Gray matter regions highlighted as outliers in the first component of surface area of the mCCA-jICA analysis of surface area and miRNA.**

| ROIs | CM < HC | CM < EM | EM > HC |
|---|---|---|---|
| L lateral occipital cortex | X | X | |
| R lateral occipital cortex | X | X | |
| L inferior parietal lobule | X | X | |
| R inferior parietal lobule | X | X | |
| L postcentral gyrus | X | X | |
| L rostral middle frontal gyrus | X | X | |
| R rostral middle frontal gyrus | X | X | |
| L superior frontal gyrus | X | X | |
| R superior frontal gyrus | X | X | |
| L superior parietal cortex | X | | X |
| R superior parietal cortex | X | | X |
| R precentral gyrus | X | X | |

| | | | |
|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | |

**Table 9. Gray matter regions highlighted as outliers in the second component of surface area of the mCCA-jICA analysis of surface area and miRNA.**

| ROIs | CM < HC | CM < EM | EM > HC |
|---|---|---|---|
| R inferior parietal lobule | X | X | |
| L rostral middle frontal gyrus | X | X | |
| R rostral middle frontal gyrus | X | X | |
| L superior frontal gyrus | X | X | |
| R superior frontal gyrus | X | X | |
| L superior parietal cortex | X | | X |
| R superior parietal cortex | X | | X |
| L supramarginal gyrus | X | X | X |

| | | | |
|---|---|---|---|
| CM = chronic migraine, EM = episodic migraine, HC = healthy control, jICA = joint independent component analysis, L = left, mCCA = multimodal canonical correlation analysis, miRNA = micro-RNA, R = right. | | | |

**Table 10. Changes in micro-RNAs (miRNAs) highlighted as outliers in the first component of miRNA of the mCCA-jICA analysis of surface area and miRNA.**

| ROI | CM < HC | EM < HC | CM < EM | CM > HC | EM > HC | CM > EM |
|---|---|---|---|---|---|---|
| miR-200a-3p | X | X | | | | |
| miR-141-3p | X | X | | | | |
| miR-375 | X | X | | | | |
| miR-454-3p | X | X | | | | |
| miR-200c-3p | X | X | | | | |
| miR-133a-3p | X | | X | | | |
| miR-483-5p | X | X | X | | | |
| miR-497-5p | X | | | | | |
| miR-335-3p | X | X | | | | |
| miR-34a-5p | | | | X | | X |
| miR-885-5p | | | | X | | X |
| miR-122-5p | | | | X | | X |
| miR-365a-3p | | | | X | | X |
| miR-126-5p | | | | X | X | |
| miR-195-5p | | | | X | | X |
| miR-136-3p | | | | X | X | |
| miR-127-3p | | | | X | | X |
| miR-376c-3p | | | | X | X | |
| miR-193a-5p | | | | X | X | X |
| miR-326 | | | | X | | |
| miR-136-5p | | | | | X | |
| miR-376a-3p | | | | | X | |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, R = right. | | | | | | |

**Table 11. Changes in micro-RNAs (miRNAs) highlighted as outliers in the second component of miRNA of the mCCA-jICA analysis of surface area and miRNA.**

| ROI | CM < HC | EM < HC | CM < EM | CM > HC | EM > HC | CM > EM |
|---|---|---|---|---|---|---|
| miR-200a-3p | X | X | | | | |
| miR-375 | X | X | | | | |
| miR-409-3p | X | | | | | |
| miR-7-5p | X | | X | | | |
| miR-485-3p | | | X | | X | |
| miR-486-5p | | | X | | X | |
| miR-451a | | | X | | X | |
| miR-136-5p | | | | | X | |
| miR-874-3p | | | | | X | |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, R = right. | | | | | | |

Regarding the secondary analysis, significant relationship between the identified miRNAs and fMRI-based connectivity was found. For the miRNA pattern that showed higher expression in CM compared to EM and HC, a relationship between the expression of miR-885-5p and miR-122-5p with higher and lower functional connectivity was detected. Specifically, lower connectivity in CM was found in all connections between the left occipital cortex, the right lateral occipital cortex, and the right cerebellum (three connections). The opposite trend with higher connectivity in CM was identified in the connections between the left postcentral and precentral gyri, and the right inferior parietal lobe and supramarginal gyrus.

Furthermore, an association with the pattern showing lower miRNA expression values in both migraine groups compared to EM and functional connectivity was observed (see details in **Tables 12-13** and **Figures 8-9**). The miRNAs related to the functional connectivity were miR-200a-3p and miR-375. Their expression was related to lower functional connectivity in the connection between the left and right rostral middle frontal gyrus, and higher functional connectivity between the left inferior parietal lobe and right caudal middle frontal gyrus.

**Table 12. Changes in micro-RNAs (miRNAs) highlighted as outliers in the second component of miRNA of the mCCA-jICA analysis of functional MRI (fMRI) and miRNA.**

| ROI | CM < HC | EM < HC | CM < EM | CM > HC | EM > HC | CM > EM |
|---|---|---|---|---|---|---|
| miR-200a-3p | X | X | | | | |
| miR-155-5p | | | | | | |
| miR-7-5p | X | | X | | | |
| miR-375 | X | X | | | | |
| miR-136-5p | | | | | X | |
| miR-1 | X | X | | | | |
| miR-885-5p | | | | X | | X |
| miR-122-5p | X | X | | | | |
| miR-483-5p | X | X | | | | |
| miR-497-5p | X | | | | | |
| miR-223-5p | | | | X | | |
| miR-193a-5p | | | | X | X | X |
| miR-335-3p | X | X | | | | |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, R = right. | | | | | | |

**Table 13. Connections between gray matter regions highlighted as outliers in the second component of functional MRI (fMRI) of the mCCA-jICA analysis of micro-RNA (miRNA) and fMRI. The regions shown were those present in the mCCA-jICA analysis between miRNA and surface area (the regions with cortical curvature differences are shown in Table 7).**

| ROIs | CM < HC | EM < HC | CM < EM | CM > HC | CM > EM |
|---|---|---|---|---|---|
| L lateral occipital cortex - R lateral occipital cortex | | | X | | |
| L lateral occipital cortex - R cerebellum | X | | X | | |
| L postcentral gyrus - R precentral gyrus | | | | X | X |
| L rostral middle frontal gyrus - R rostral middle frontal gyrus | X | X | | | |
| R inferior parietal lobe - R supramarginal gyrus | X | | | | X |
| R lateral occipital cortex - R cerebellum | X | | | | |

| | | | | | |
|---|---|---|---|---|---|
| CM = chronic migraine, EM = episodic migraine, HC = healthy control, jICA = joint independent component analysis, L = left, mCCA = multimodal canonical correlation analysis, R = right. | | | | | |

### Example 2.4. MiRNA and gray matter volume

Two components showed statistically significant differences between the groups. On the one hand, the second gray matter volume component showed lower mixing coefficient values in CM compared to EM (pANOVA = 0.022, pTukey = 0.023) and it was correlated with the corresponding miRNA component (r = 0.40, p = 0.028). In relation to this result, the second miRNA component outliers showed lower expression in CM compared to EM. This set of changes presented no clear relationship with any of the three main patterns of miRNA changes. On the other hand, the third miRNA component showed higher mixing coefficient values in CM compared to HC (pANOVA = 0.038, pTukey = 0.040), but it showed no correlation with the corresponding volume component. In this component, after the assessment of outliers, the three described patterns of changes in miRNA expression were identified. The specific miRNAs and regions that were classified as outliers, together with the distribution and correlation plot of the mixing coefficients with statistically significant differences, are shown in **Tables 14-16** and **Figures 10-11****.**

**Table 14. Gray matter regions highlighted as outliers in the second component of gray matter volume of the mCCA-jICA analysis of gray matter volume and micro-RNA (miRNA).**

| ROIs | CM < HC | CM < EM |
|---|---|---|
| R inferior parietal lobule | | X |
| L inferior temporal gyrus | | X |
| L lateral occipital cortex | X | X |
| R lateral occipital cortex | X | X |
| L middle temporal gyrus | X | X |
| L rostral middle frontal gyrus | X | X |
| R rostral middle frontal gyrus | X | X |
| L superior frontal gyrus | X | X |
| R superior frontal gyrus | X | X |
| L cerebellum | X | X |
| R cerebellum | X | X |
| R superior parietal cortex | X | X |

| | | |
|---|---|---|
| CM = chronic migraine, EM = episodic migraine, HC = healthy control, jICA = joint independent component analysis, L = left, mCCA = multimodal canonical correlation analysis, R = right. | | |

**Table 15. Micro-RNAs (miRNAs) highlighted as outliers in the second component of miRNA of the mCCA-jICA analysis of gray matter volume and miRNA.**

| miRNA | CM < HC | EM < HC | CM < EM | CM > HC | EM > HC | CM > EM |
|---|---|---|---|---|---|---|
| miR-486-5p | | | | X | | X |
| miR-451a | | | X | | X | |
| miR-409-3p | X | | | | | |
| miR-485-3p | | | X | | X | |
| miR-497-5p | X | | | | | |
| miR-100-5p | | X | | | | X |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, R = right. | | | | | | |

**Table 16. Micro-RNAs (miRNAs) highlighted as outliers in the third component of miRNA of the mCCA-jICA analysis of gray matter volume and miRNA.**

| ROI | CM < HC | EM < HC | CM < EM | CM > HC | EM > HC | CM > EM |
|---|---|---|---|---|---|---|
| miR-200a-3p | X | X | | | | |
| miR-375 | X | X | | | | |
| miR-1 | X | X | | | | |
| miR-7-5p | X | | X | | | |
| miR-483-5p | X | X | X | | | |
| miR-497-5p | X | | | | | |
| miR-335-3p | X | X | | | | |
| miR-34a-5p | | | | X | | X |
| miR-885-5p | | | | X | | X |
| miR-122-5p | | | | X | | X |
| miR-365a-3p | | | | X | | X |
| miR-126-5p | | | | X | X | |
| miR-195-5p | | | | X | | X |
| miR-99a-5p | | | | X | | X |
| miR-125b-5p | | | | X | | X |
| miR-376c-3p | | | | X | X | |
| miR-193a-5p | | | | X | X | X |
| miR-194-5p | | | | X | | X |
| miR-136-5p | | | | | X | |
| miR-376a-3p | | | | | X | |

| | | | | | | |
|---|---|---|---|---|---|---|
| CM = chronic migraine, EM = episodic migraine, HC = healthy control, jICA = joint independent component analysis, L = left, mCCA = multimodal canonical correlation analysis, R = right. | | | | | | |

### Example 2.5. MiRNA and brain MRI signatures

Regarding the statistical comparisons of the miRNAs between the three groups, three miRNAs showed statistically significant differences between EM or CM and HC, showing all of them lower values in the migraine group compared to HC: miR-375 (pKruskal-Wallis = 0.035, pConover-Iman = 0.005 for EM < HC), miR-454-3p (pANOVA= 0.006, pTukey= 0.006 for CM < HC, pTukey= 0.041 for EM < HC), and miR-16-2-3p (pKruskal-Wallis = 0.046, pConover-Iman = 0.007 for CM < HC). Two additional miRNAs that showed lower expression in CM and EM according to the joint miRNA-SA analysis (**Figure 5**) were considered: miR-200a-3p and miR-141-3p. The univariate AUC values from the logistic regression analysis are shown in **Table 17.** Briefly, miR-375 showed the most stables AUC values for the classification of any migraine group or all the migraine patients against HC (AUC between 0.77 and 0.80). For the classification of EM and CM patients, all miRNAs showed AUC values around 0.60, except miRNA-200a-3p, with an AUC value of 0.69.

**Table 17. Area under the curve (AUC) values of the univariate selected miRNAs for migraine diagnosis.**

| miRNA | Migraine vs. HC | EM vs. HC | CM vs. HC | CM vs. EM |
|---|---|---|---|---|
| miR-375 | 0.785 | 0.80 | 0.77 | 0.61 |
| miR-200a-3p | 0.59 | 0.53 | 0.71 | 0.69 |
| miR-141-3p | 0.725 | 0.71 | 0.74 | 0.59 |
| miR-454-3p | 0.80 | 0.76 | 0.84 | 0.60 |
| miR-16-2-3p | 0.77 | 0.73 | 0.81 | 0.60 |

Considering the stable results of miR-375, and that this miRNA was the only present in the statistical comparisons and the mCCA-jICA procedure, it was the reference miRNA for the bivariate assessments. The bivariate AUC values from the logistic regression models combining miR-375 with the remaining miRNAs are shown in **Table 18.** The combination of miR-375 and miR-200a-3p showed the highest AUC values for the classification of EM and HC (AUC=0.86), and CM vs EM (AUC=0.73). The combination of miR-375 and miR-454-3p showed the best overall migraine (AUC=0.87) and CM and HC (AUC=0.90) classification results.

**Table 18. Area under the curve (AUC) values of the bivariate selected miRNAs for migraine diagnosis.**

| miRNA | Migraine vs. HC | EM vs. HC | CM vs. HC | CM vs. EM |
|---|---|---|---|---|
| miR-375 + miR-200a-3p | 0.815 | 0.86 | 0.76 | 0.73 |
| miR-375 + miR-141-3p | 0.785 | 0.80 | 0.77 | 0.58 |
| miR-375 + miR-454-3p | 0.87 | 0.84 | 0.90 | 0.62 |
| miR-375 + miR-16-2-3p | 0.795 | 0.84 | 0.87 | 0.63 |

When combining the gray matter morphometry with the five miRNAs, the classification for CM, considering the classification with EM and HC, improved in all cases compared to the univariate and the bivariate miRNA models. Specifically, the surface area of the right superior frontal gyrus was taken as an example as it provided global decent results for all considered scenarios. **Table 19** shows the AUC values for the combination of the five miRNAs and the mentioned area parameter. The combination of miR-375 with the area was the only option with AUC values over 0.80 for all classifications, providing the best AUC of the classification between EM and HC (AUC=0.81). The combination with miR-454-3p provided the best AUC for all migraine (AUC=0.87) and CM patients (AUC=0.98), and the combination with miR-16-2-3p the best AUC for the classification between CM and EM (AUC=0.96).

**Table 19. Area under the curve (AUC) values of the univariate selected miRNAs combined with the surface area of the right superior frontal gyrus (R-SFG) for migraine diagnosis.**

| miRNA | Migraine vs. HC | EM vs. HC | CM vs. HC | CM vs. EM |
|---|---|---|---|---|
| miR-375 + area R-SFG | 0.825 | 0.81 | 0.92 | 0.87 |
| miR-200a-3p + area R-SFG | 0.705 | 0.56 | 0.89 | 0.89 |
| miR-141-3p + area R-SFG | 0.77 | 0.65 | 0.92 | 0.91 |
| miR-454-3p + area R-SFG | 0.87 | 0.79 | 0.98 | 0.89 |
| miR-16-2-3p + area R-SFG | 0.645 | 0.56 | 0.90 | 0.96 |

Finally, for the combination of the bivariate miRNA with surface area logistic regression models, all options presented AUC values over 0.8 except the classification between EM and HC for miR-375, miR-141-3p and the area (AUC=0.76). All the AUC values are shown in **Table 20.** In line with the univariate miRNA combined with area assessments, the combination of the area and miR-375 with miR-200a-3p showed the best AUC for the classification between EM and HC (AUC=0.86), with miR-454-3p for overall migraine (AUC=0.91) and CM and HC (AUC=0.97), and with miR-16-2-3p for classification between CM and EM (AUC=0.97).

It is worth noting that the number of variables was restricted to a maximum of three to avoid a number of variables higher than a 10% of the total sample size.

**Table 20. Area under the curve (AUC) values of the bivariate selected miRNAs combined with the surface area of the right superior frontal gyrus (R-SFG) for migraine diagnosis.**

| miRNA | Migraine vs. HC | EM vs. HC | CM vs. HC | CM vs. EM |
|---|---|---|---|---|
| miR-375 + miR-200a-3p + area R-SFG | 0.845 | 0.86 | 0.91 | 0.89 |
| miR-375 + miR-141-3p + area R-SFG | 0.815 | 0.76 | 0.91 | 0.91 |
| miR-375 + miR-454-3p + area R-SFG | 0.91 | 0.85 | 0.97 | 0.89 |
| miR-375 + miR-16-2-3p + area R-SFG | 0.83 | 0.82 | 0.96 | 0.97 |

### Example 2.6. Extraction of unsupervised clusters

The optimal number of clusters according to the majority rule was 3. It was appreciated that one of the clusters contained two outliers. Hence, the analysis was repeated with two clusters. For k-means and hierarchical clustering, the extracted clusters were divided with the same number of subjects per group. In the clustering including HC, EM and CM one of the clusters was composed of 4 HCs, 1 patient with EM and all patients with CM (10), while the other one included the remaining subjects. i.e., 6 HCs, 9 patients with EM and no CMs. In the clustering including HC, EM and CM one of the clusters was composed of 9 EM and the other cluster was composed by 10 CM and 1 EM. The clusters and dendrogram obtained with the hierarchical clustering are shown in **Figure 13** (HC, EM and CM) and **16** (EM and CM), and the k-means results, the original assessment with three clusters, the analysis of the optimal number of clusters and the silhouette profile of the two extracted clusters are available in **Figures 13-18****.**

### References:

Andersson, J. L. R., & Sotiropoulos, S. N. (2016). An integrated approach to correction for off-resonance effects and subject movement in diffusion MR imaging. Neurolmage, 125, 1063-1078. https://doi.org/10.1016/j.neuroimage.2015.10.019
Andreu, Z., Rivas, E., Sanguino-Pascual, A., Lamana, A., Marazuela, M., González-Alvaro, I., Sánchez-Madrid, F., de la Fuente, H., & Yáñez-Mó, M. (2016). Comparative analysis of EV isolation procedures for miRNAs detection in serum samples. Journal of Extracellular Vesicles, 5(1), 31655. https://doi.org/10.3402/jev.v5.31655
Charrad, M., Ghazzali, N., Boiteau, V., & Niknafs, A. (2014). NbClust: An R Package for Determining the Relevant Number of Clusters in a Data Set. Journal of Statistical Software, 61, 1-36. https://doi.org/10.18637/jss.v061.i06
Cordero-Grande, L., Christiaens, D., Hutter, J., Price, A. N., & Hajnal, J. V. (2019). Complex diffusion-weighted image estimation via matrix recovery under general noise models. Neurolmage, 200, 391-404. https://doi.org/10.1016/j.neuroimage.2019.06.039
Desikan, R. S., Ségonne, F., Fischl, B., Quinn, B. T., Dickerson, B. C., Blacker, D., Buckner, R. L., Dale, A. M., Maguire, R. P., Hyman, B. T., Albert, M. S., & Killiany, R. J. (2006). An automated labeling system for subdividing the human cerebral cortex on MRI scans into gyral based regions of interest. Neurolmage, 31(3), 968-980. https://doi.org/10.1016/j.neuroimage.2006.01.021
Headache Classification Committee of the International Headache Society (IHS) The International Classification of Headache Disorders, 3rd edition. (2018). Cephalalgia, 38(1), 1-211. https://doi.org/10.1177/0333102417738202
Horn, J. L. (1965). A rationale and test for the number of factors in factor analysis. Psychometrika, 30(2), 179-185. https://doi.org/10.1007BF02289447
Kellner, E., Dhital, B., Kiselev, V. G., & Reisert, M. (2016). Gibbs-ringing artifact removal based on local subvoxel-shifts. Magnetic Resonance in Medicine, 76(5), 1574-1581. https://doi.org/10.1002/mrm.26054
Oishi, K., Zilles, K., Amunts, K., Faria, A., Jiang, H., Li, X., Akhter, K., Hua, K., Woods, R., Toga, A. W., Pike, G. B., Rosa-Neto, P., Evans, A., Zhang, J., Huang, H., Miller, M. I., van Zijl, P. C. M., Mazziotta, J., & Mori, S. (2008). Human brain white matter atlas: Identification and assignment of common anatomical structures in superficial white matter. NeuroImage, 43(3), 447-457. https://doi.org/10.1016/j.neuroimage.2008.07.009
Planchuelo-Gómez, Á., Garcia-Azorin, D., Guerrero, Á. L., Aja-Fernández, S., Rodriguez, M., & de Luis-García, R. (2021). Multimodal fusion analysis of structural connectivity and gray matter morphology in migraine. Human Brain Mapping, 42(4), 908-921. https://doi.org/10.1002/hbm.25267
Rousseeuw, P. J. (1987). Silhouettes: A graphical aid to the interpretation and validation of cluster analysis. Journal of Computational and Applied Mathematics, 20, 53-65. https://doi.org/10.1016/0377-0427(87)90125-7
Smith, S. M., Jenkinson, M., Woolrich, M. W., Beckmann, C. F., Behrens, T. E. J., Johansen-Berg, H., Bannister, P. R., De Luca, M., Drobnjak, I., Flitney, D. E., Niazy, R. K., Saunders, J., Vickers, J., Zhang, Y., De Stefano, N., Brady, J. M., & Matthews, P. M. (2004). Advances in functional and structural MR image analysis and implementation as FSL. NeuroImage, 23 Suppl 1, S208-219. https://doi.org/10.1016/j.neuroimage.2004.07.051
Tournier, J.-D., Smith, R., Raffelt, D., Tabbara, R., Dhollander, T., Pietsch, M., Christiaens, D., Jeurissen, B., Yeh, C.-H., & Connelly, A. (2019). MRtrix3: A fast, flexible and open software framework for medical image processing and visualisation. NeuroImage, 202, 116137. https://doi.org/10.1016/j.neuroimage.2019.116137
Veraart, J., Fieremans, E., Jelescu, I. O., Knoll, F., & Novikov, D. S. (2016). Gibbs ringing in diffusion MRI. Magnetic Resonance in Medicine, 76(1), 301-314. https://doi.org/10.1002/mrm.25866
Zhang, Y., Brady, M., & Smith, S. (2001). Segmentation of brain MR images through a hidden Markov random field model and the expectation-maximization algorithm. IEEE Transactions on Medical Imaging, 20(1), 45-57. https://doi.org/10.1109/42.906424

## Claims

1. *In vitro* method for the diagnosis of migraine which comprises: (a) assessing the expression level of miR-375 in a biological sample obtained from a subject, b) wherein the identification of a lower expression level, as compared with a pre-established threshold value determined in subjects that do not suffer from migraine or by comparing subjects that suffer from migraine with subjects that do not suffer from migraine, is an indication that the subject suffers from migraine.

2. *In vitro* method for the diagnosis of migraine, according to claim 1, which comprises: (a) assessing the expression level of miR-375 in combination with the expression level of a miRNA selected from: miR-200a-3p, miR-141-3p, miR-454-3p and/or miR-16-2-3p in a biological sample obtained from a subject, b) wherein the identification of a lower expression level, as compared with a pre-established threshold value determined in subjects that do not suffer from migraine or by comparing subjects that suffer from migraine with subjects that do not suffer from migraine, is an indication that the subject suffers from migraine.

3. *In vitro* method for the diagnosis of migraine, according to any of the claims 1 or 2, which comprises: (a) assessing the expression level of the following combinations of miRNAs: miR-375 and miR-200a-3p, miR-375 and miR-454-3p; and/or miR-375 and miR-16-2-3p in a biological sample obtained from a subject, b) wherein the identification of a lower expression level of the combination of miR-375 and miR-200a-3p, miR-375 and miR-454-3p; and/or miR-375 and miR-16-2-3p, as compared with a pre-established threshold value determined in subjects that do not suffer from migraine or by comparing subjects that suffer from migraine with subjects that do not suffer from migraine, is an indication that the subject suffers from migraine.

4. *In vitro* method for the diagnosis of migraine, according to any of the claims 1 or 3, which further comprises assessing the area of the right superior frontal gyrus, preferably by magnetic resonance imaging (MRI), wherein a lower surface area of the right superior frontal gyrus, as compared with a pre-established threshold value determined in subjects that do not suffer from migraine or by comparing subjects that suffer from migraine with subjects that do not suffer from migraine, is an indication that the subject suffers from migraine.

5. *In vitro* method for the diagnosis of migraine, according to any of the claims 1 or 4, wherein the migraine is chronic migraine or episodic migraine.

6. *In vitro* method for the differential diagnosis of chronic and episodic migraine which comprises: (a) assessing the expression level of miR-375 in a biological sample obtained from a subject, b) wherein the identification of a lower expression level, as compared with a pre-established threshold value determined in subjects that suffer from episodic migraine or by comparing subjects that suffer from episodic and chronic migraine, is an indication that the subject suffers from chronic migraine, and c) wherein the identification of a higher expression level, as compared with a pre-established threshold value determined in subjects that suffer from chronic migraine or by comparing subjects that suffer from episodic and chronic migraine, is an indication that the subject suffers from episodic migraine.

7. *In vitro* method for the differential diagnosis of chronic and episodic migraine, according to claim 6, which comprises: (a) assessing the expression level of miR-375 in combination with the expression level of a miRNA selected from: miR-200a-3p, miR-141-3p, miR-454-3p and/or miR-16-2-3p in a biological sample obtained from a subject, b) wherein the identification of a lower expression level, as compared with a pre-established threshold value determined in subjects that suffer from episodic migraine or by comparing subjects that suffer from episodic and chronic migraine, is an indication that the subject suffers from chronic migraine, and c) wherein the identification of a higher expression level, as compared with a pre-established threshold value determined in subjects that suffer from chronic migraine or by comparing subjects that suffer from episodic and chronic migraine, is an indication that the subject suffers from episodic migraine.

8. *In vitro* method for the differential diagnosis of chronic and episodic migraine, according to any of the claims 6 or 7, which comprises: a) assessing the expression level of the following combinations of miRNAs: miR-375 and miR-200a-3p, miR-375 and miR-454-3p; and/or miR-375 and miR-16-2-3p in a biological sample obtained from a subject, b) wherein the identification of a lower expression level, as compared with a pre-established threshold value determined in subjects that suffer from episodic migraine or by comparing subjects that suffer from episodic and chronic migraine, is an indication that the subject suffers from chronic migraine, and c) wherein the identification of a higher expression level, as compared with a pre-established threshold value determined in subjects that suffer from chronic migraine or by comparing subjects that suffer from episodic and chronic migraine, is an indication that the subject suffers from episodic migraine.

9. *In vitro* method for the differential diagnosis of chronic an episodic migraine, according to any of the claims 6 to 8, which further comprises: a) assessing the area of the right superior frontal gyrus by magnetic resonance imaging (MRI) of the subject, b) wherein a lower surface area of the right superior frontal gyrus, as compared with a pre-established threshold value determined in subjects that suffer from episodic migraine or by comparing subjects that suffer from episodic and chronic migraine, is an indication that the subject suffers from chronic migraine, and c) wherein a higher surface area of the right superior frontal gyrus, as compared with a pre-established threshold value determined in subjects that suffer from chronic migraine or by comparing subjects that suffer from episodic and chronic migraine, is an indication that the subject suffers from episodic migraine.

10. *In vitro* method, according to any of the previous claims, wherein the biological sample is selected from serum-derived extracellular vesicles, plasma-derived extracellular vesicles, blood-derived extracellular vesicles, blood, serum or plasma, preferably wherein the biological sample is serum-derived extracellular vesicles.

11. *In vitro* use of the expression level of miR-375 for the diagnosis of migraine, or for the differential diagnosis of chronic and episodic migraine.

12. *In vitro* use, according to claim 11, of the expression level of miR-375 in combination with the expression level of a miRNA selected from: miR-200a-3p, miR-141-3p, miR-454-3p and/or miR-16-2-3p for the diagnosis of migraine, or for the differential diagnosis of chronic and episodic migraine.

13. *In vitro* use, according to any of the claims 11 or 12, of the expression level of the following combinations of miRNA: miR-375 and miR-200a-3p, miR-375 and miR-454-3p, and/or miR-375 and miR-16-2-3p for the diagnosis of migraine, or for the differential diagnosis of chronic and episodic migraine.

14. *In vitro* use, according to any of the claims 11 to 13, in combination with the assessment the area of the right superior frontal gyrus by magnetic resonance imaging (MRI).

15. *In vitro* use, according to claims 11 to 14, wherein the diagnosis of migraine is the diagnosis of chronic or episodic migraine.
